(19) Europäisches Patentamt
European Patent Office
Office européen des brevets

(11) Veröffentlichungsnummer : **0 183 193**
**B1**

(12) # EUROPÄISCHE PATENTSCHRIFT

(45) Veröffentlichungstag der Patentschrift : **24.01.90**

(21) Anmeldenummer : **85114797.5**

(22) Anmeldetag : **21.11.85**

(51) Int. Cl.$^5$ : **C 07 D403/06**, C 07 F 3/00, C 07 F 1/00, C 07 F 15/04, C 07 F 15/06, C 09 B 57/00, C 08 K 5/00

(54) **Neue Isoindolinverbindungen, deren Metallkomplexe und deren Verwendung.**

(30) Priorität : **29.11.84 DE 3443465**

(43) Veröffentlichungstag der Anmeldung : **04.06.86 Patentblatt 86/23**

(45) Bekanntmachung des Hinweises auf die Patenterteilung : **24.01.90 Patentblatt 90/04**

(84) Benannte Vertragsstaaten : **CH DE FR GB IT LI**

(56) Entgegenhaltungen :
**DE-A- 2 359 791**
**DE-A- 3 208 218**
**DE-A- 3 311 375**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Lotsch, Wolfgang, Dr.**
**Stettiner Strasse 32**
**D-6711 Beindersheim (DE)**

**EP 0 183 193 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Metallkomplexe von Isoindolinverbindungen, die durch die Formel (II)

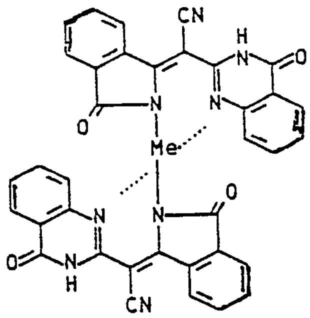

(II)

gekennzeichnet sind, worin Me für ein zweiwertiges Kobalt- oder Nickelion steht.

Die neuen Verbindungen (II) geben grünstichig gelbe bis rotstichig gelbe Färbungen.

Die neuen Farbstoffe stellen wertvolle Pigmente dar, welche in feinverteilter Form zum Pigmentieren von hochmolekularem organischem Material verwendet werden können. Als solches kommt z. B. in Betracht: Celluloseether und -ester, wie Ethylcellulose, Nitrocellulose, Celluloseacetat, Cellulosebutyrat; natürliche Harze oder Kunstharze, wie Polymerisationsharze oder Kondensationsharze, z. B. Aminoplaste, insbesondere Harnstoff- und Melamin-Formaldehydharze, Alkydharze, Phenoplaste, Polycarbonate, Polyolefine, z. B. Polystyrol, Polyvinylchlorid, Polyethylen, Polypropylen, Polyacrylnitril, Polyacrylsäureester; Polyamide, Polyurethane oder Polyester; Gummi, Casein, Silikon und Silikonharze, allein oder in Mischungen.

Dabei spielt es keine Rolle, ob die erwähnten hochmolekularen Verbindungen als plastische Massen, Schmelzen oder in Form von Spinnlösungen, Lacken, Anstrichstoffen oder Druckfarben vorliegen. Je nach Verwendungszweck erweist es sich als vorteilhaft, die neuen Pigmente als Toner oder in Form von Präparaten zu verwenden. Um fein verteilte Pigmente zu erreichen, ist es oft von Vorteil, diese einem Mahlprozess zu unterwerfen.

Die Komplexe (II) eignen sich außer zum Färben von Thermoplasten in der Masse auch zum Pigmentieren von Druckfarben und Lacken.

Gegenüber Färbungen, die mit Metallkomplexen der DE-OS 23 59 791 hergestellt wurden, liefern die Metallkomplexe der Erfindung Färbungen mit höherer Reinheit im Farbton und höherer Farbstärke.

Me steht für zweiwertige Ionen von Kobalt und Nickel, insbesondere für solche des Kobalts.

Die den Komplexen zugrundeliegende Isoindolinverbindung wird in an sich bekannter Weise durch Kondensation von 2-Cyanmethyl-chinazolon (III)

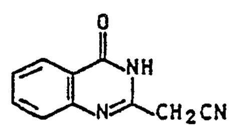

(III)

mit einer Isoindolinonverbindung der Formel (IV)

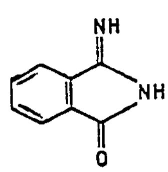

(IV)

erhalten.

Zweckmäßigerweise erfolgt die Kondensation in einem organischen Lösungsmittel, z. B. in Alkoholen wie Methanol, Ethanol, Isopropanol, Ethylenglykolmonoethylether, Diethylenglykolmonoethylether oder in Eisessig, Propionsäure oder Dimethylformamid bei erhöhter Temperatur.

Da die Kondensationsprodukte in den genannten Mitteln schwer löslich sind, können erstere leicht durch Filtrieren isoliert werden. Gegebenenfalls vorhandene Verunreinigungen können durch Waschen entfernt werden.

Zur Überführung in (II) werden die Kondensationsprodukte mit Mitteln behandelt, welche zweiwertige Kationen des Nickels oder Kobalts abgeben.

Vorzugsweise verwendet man die Formiate, Acetate oder auch Acetylacetonate dieser Metalle, z. B. Nickel-II-acetat oder Kobalt-II-acetat.

Die Metallisierung erfolgt vorteilhafterweise in einem oder in einem Gemisch der oben angegebenen Lösungsmittel. Als Lösungsmittel sind Dimethylformamid und Diethylenglykolmonoethylether bevorzugt.

Es ist auch möglich die Kondensation und die Metallisierung im Eintopfverfahren durchzuführen.

Die Erfindung wird durch die folgenden Ausführungsbeispiele weiter erläutert.

Die im folgenden angegebenen Teile sind Gewichtsteile, die Prozente Gewichtsprozente.

Beispiel 1

19 Teile 2-Cyanmethyl-chinazolon und 19 Teile Iminophthalimid-hydrochlorid wurden in 150 Teilen Eisessig unter Rühren auf 120 °C erwärmt und 5 Stunden bei dieser Temperatur gehalten. Nach dem Erkalten wurde der Niederschlag abgesaugt, mit Methanol und Wasser gewaschen und im Vakuum bei 80 bis 100 °C getrocknet. Ausbeute : 25 Teile Kondensationsprodukt.

Die Verbindung gibt bei Massefärbung in Polystyrol reine grünstichig gelbe Färbungen mit guter Lichtechtheit.

Beispiel 2

32 Teile der nach Beispiel 1 erhaltenen Verbindung wurden in 300 Teilen Dimethylformamid nach der Zugabe von 26 Teilen Nickelacetat $\cdot$ $4H_2O$ 16 Stunden bei 150 °C gerührt. Der Niederschlag wurde heiß abgesaugt, mit Methanol und Wasser gewaschen. Es wurde ein grünstichig gelbes Pigmentpulver erhalten. Ausbeute : 26 Teile. Beim Einwalzen in Polyvinylchlorid wurde eine gelbe Folie erhalten, die gute Migrations- und Lichtechtheit besitzt. Ein mit dem erhaltenen Nickelkomplex pigmentierter Lack zeigte sehr gute Licht- und Wetterbeständigkeit.

Beispiel 3

Es wurde das in Beispiel 2 verwendete Nickelacetat durch die äquimolare Menge Cobaltacetat $\cdot$ $4H_2O$ ersetzt. Es wurde ein rotstichig gelber Komplex erhalten.

Anwendungsbeispiele

a) Lack

10 Teile des nach Beispiel 2 erhaltenen Farbstoffes und 95 Teile Einbrennlackmischung, die 70 % Kokosalkydharz (60 %ig in Xylol gelöst) und 30 % Melaminharz (ungefähr 55 %ig gelöst in Butanol/Xylol) enthält, werden in einem Attritor angerieben. Nach dem Auftragen und einer Einbrennzeit von 30 Minuten bei 120 °C werden grünstichig gelbe Volltonlackierungen mit guter Licht- und Überspritzechtheit erhalten. Durch Zumischen von Titandioxid werden gelbe Färbungen erhalten.

Verwendet man den in Beispiel 3 beschriebenen Farbstoff, so werden Lackierungen in rotstichig gelbem Farbton und ähnlichen anwendungstechnischen Eigenschaften erhalten.

b) Kunststoff

0,5 Teile des nach Beispiel 2 erhaltenen Farbstoffs werden auf 100 Teile Polystyrolgranulat (Standard-Marke) aufgetrommelt. Das angefärbte Granulat wird durch Extrudieren homogenisiert (190 bis 195 °C). Man erhält grünstichig gelbe Extrudate, deren Färbung gute Lichtechtheit aufweist.

Verwendet man Mischungen aus 0,5 Teilen Farbstoff und 1 Teil Titandioxid, so erhält man ebenfalls gelbe Färbungen.

Verwendet man den Pigmentfarbstoff des Beispiels 3, so erhält man rotstichige Gelbfärbungen mit guten Lichtechtheiten.

## Patentansprüche

1. Metallkomplexe von Isoindolinverbindungen, gekennzeichnet durch die Formel (II)

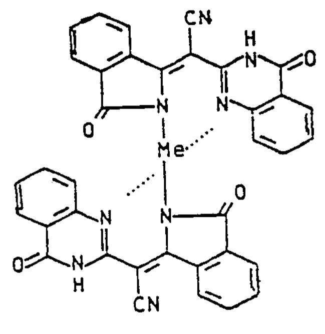

(II)

in der Me für ein zweiwertiges Kobalt- oder Nickelion steht.

2. Verwendung der Metallkomplexe gemäß Anspruch 1 zum Pigmentieren von hochmolekularen Materialien.

## Claims

1. A metal complex of an isoindoline compound, which is of the formula (II)

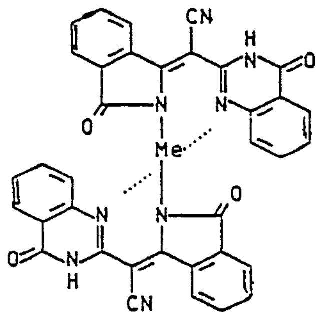

(II)

where Me is a divalent cobalt or nickel ion.

2. The use of a metal complex as claimed in claim 1 for pigmenting high molecular weight materials.

## Revendications

1. Complexes métalliques de dérivés d'isoindoline, caractérisés par la formule II

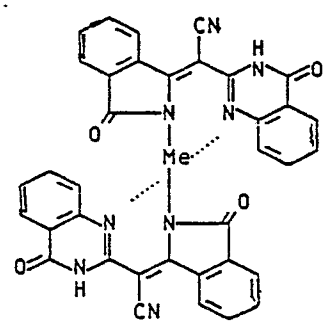

(II)

dans laquelle Me est mis pour un ion cobalt ou nickel bivalent.

2. Utilisation des complexes métalliques selon la revendication 1, pour la pigmentation de matériaux à haut poids moléculaire.